# EUROPEAN PATENT APPLICATION

(11) **EP 4 342 385 A1**
(43) Date of publication of application: **27.03.2024**
(21) Application number: 22197657.4
(22) Date of filing: 26.09.2022
(51) Int. Cl.: A61B 6/10

(54) **MEDICAL DEVICE MOVEMENT CONTROL APPARATUS**

(71) Applicant: Koninklijke Philips N.V., 5656 AG Eindhoven (NL)
(72) Inventor: JAMODKAR, Abhijeet Dnyaneshwar, Eindhoven (NL); MANDE, Vishal, Eindhoven (NL)
(74) Representative: Philips Intellectual Property & Standards

(57) **Abstract**

The present invention relates to a medical device movement control apparatus (10), comprising:
- an input unit (20);
- at least one distance sensor (30); and
- a controller (40);
wherein the input unit is configured to receive a required location of the medical device;
wherein the input unit is configured to provide the required location of the medical device to the controller;
wherein the at least one distance sensor is configured to be mounted to a medical device (50) or is configured to be integrated with the medical device;
wherein the at least one distance sensor is configured to acquire distance data from the medical device for a plurality of angular directions about the medical device;
wherein the at least one distance sensor is configured to provide the distance data for the plurality of angular directions about the medical device to the controller; and
wherein the controller is configured to control a movement system (60) of the medical device to move the medical device from an initial location of the medical device to the required location along a route between the initial location and the required location that maintains a distance of at least a guard range distance between the medical device and one or more of objects comprising utilization of distance data for a subset of the plurality of angular directions acquired at a plurality of locations along the route.

## Description

### FIELD OF THE INVENTION

The present invention relates to a medical device movement control apparatus, a medical device movement control system, a medical device movement control method, a computer program element, and a computer readable medium.

### BACKGROUND OF THE INVENTION

Many X-ray systems incorporate the motion of X-ray tube heads and/or X-ray detectors, which are heavy and can pose a risk of colliding with patients.

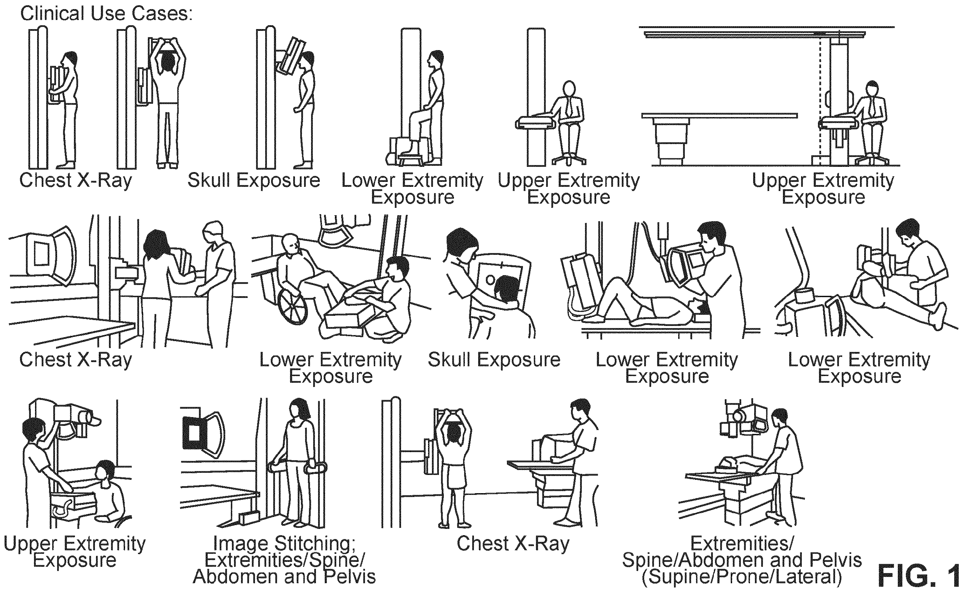

Fig. 1 shows various clinical uses cases of X-ray system, such as a Philips C90 system or Philips Azurion. In Fig 1: "Chest X-ray" is signified by "A", "Skull exposure" is signified by "B", "Lower extremity exposure" is signified by "C", "Upper extremity exposure" is signified by "D", "Image stitching, extremities/spine/abdomen and Pelvis" is signified by "E", and "Extremities/spine/abdomen and pelvis (Supine/prone/lateral)" is signified by "F". These are just exemplar use cases, and X-ray systems can be utilized in multitudinous ways, for which the present medical device movement control apparatus, system and method finds applicability.

As there are serious consequences concerning the X-ray tube head and/or X-ray detector hitting people and/or other objects, collision detection system have been developed that bring for example the X-ray tube head to a stop if a collision is detected.

One developed system detects a collision after a contact/collision between the X-ray tube head and a patient has happened and restricts movement of the X-ray tube head thereafter to avoid damage/further damage to the patient.

Other systems detect the possibility of a collision between the X-ray tube head and a patient in advance and restrict movement of the X-ray tube head to avoid damage/collision to/with the patient.

However, such developed systems can still collide with people and objects and cause damage, and even if the X-ray tube head or X-ray detector is stopped before collision, it then takes time to then re-start movement of the X-ray tube head or X-ray detector to a required location.

There is a need to address these problems.

### SUMMARY OF THE INVENTION

It would be advantageous to have an improved technique for moving a medical device such as an X-ray tube head or X-ray detector. The object of the present invention is solved with the subject matter of the independent claims, wherein further embodiments are incorporated in the dependent claims.

In a first aspect, there is provided a medical device movement control apparatus, comprising:
- an input unit;
- at least one distance sensor; and
- a controller.

The input unit is configured to receive a required location of the medical device. The input unit is configured to provide the required location of the medical device to the controller. The at least one distance sensor is configured to be mounted to a medical device or is configured to be integrated with the medical device. The at least one distance sensor is configured to acquire distance data from the medical device for a plurality of angular directions about the medical device. The at least one distance sensor is configured to provide the distance data for the plurality of angular directions about the medical device to the controller. The controller is configured to control a movement system of the medical device to move the medical device from an initial location of the medical device to the required location along a route between the initial location and the required location that maintains a distance of at least a guard range distance between the medical device and one or more of objects comprising utilization of distance data for a subset of the plurality of angular directions acquired at a plurality of locations along the route.

In this way, a medical device such as an X-ray tube head or an X-ray detector of an X-ray system can be automatically moved continuously to a required location, and moved around objects that are in the way in moving to the final location, but automatically stop if an object, that could itself be moving such as a person, gets too close to the X-ray tube head or X-ray detector.

The apparatus can for example be retro-fitted to an existing medical device such as an X-ray tube head or X-ray detector of an X-ray system, such as an X-ray attenuation examination systems, a digital radiography system, a fluoroscopy system.

Taking the example of an X-ray system with an X-ray tube head and an X-ray detector, the apparatus can autonomously control movement of the X-ray tube head to a required location of the X-ray tube head, or can autonomously control movement of the X-ray detector to a required location of the X-ray detector location, where distances sensor(s) can be mounted to or integrated with the X-ray tube head or X-ray detector respectively. However, distance sensors can be mounted to or integrated with the X-ray tube head and also with the X-ray detector, and a single controller can move the X-ray tube head to a required location of the X-ray tube head and move the X-ray detector to a required location of the X-ray detector, for example either side of a body part of a person.

In an example, the controller is configured to control the movement system of the medical device to bring the medical device to a halt if distance data for an object indicates that it is closer to the medical device than the guard range distance comprising utilization of distance data for the plurality of angular directions about the medical device at one or more of the plurality of locations along the route.

In an example, the distance data from the medical device acquired by the at least one sensor for the plurality of angular directions comprises distance data up to a threshold distance range that is greater than the guard range distance and less than a maximum detection range of the at least one distance sensor.

In an example, the threshold is 0.5m, 1m, 1.5m, 2m, 2.5m, 3m, 3.5m.

Thus, rather than acquire actual distance data in all directions, the distance sensor only passes distance information within a threshold range to the controller, for example only distance data for objects that are within a range of 2m of medical device is provided to the controller, where in the direction of a wall that is 4m away the data for be set at 2m, however for objects within the threshold range of 2m actual range data, for example 1.78m would be provided to the controller. This provides for improved computational efficiency.

It is to be noted that the threshold can be less than 0.5m, between 0.5 to 1m, between 1 to 1.5m, between 1.5m to 2m, between 2 to 2.5m, between 2.5 to 3m, between 3 to 3.5m and also greater than 3m.

In an example, the plurality of angular directions comprises a plurality of angular directions in a horizontal plane.

In an example, the subset of the plurality of angular directions at each of the plurality of locations along the route comprises angular directions over an angular range either side of a direct line between the medical device and the required location at each of the plurality of locations.

In an example, the angular range either side of the direct line between the medical device and the required location at each of the plurality of locations comprises an angle in the horizontal plane of 10 degrees, 15 degrees, 20 degrees, 25 degrees, 30 degrees, 35 degrees, 40 degrees, 45 degrees, 50 degrees, 55 degrees, 60 degrees.

Thus, the at least one sensor can acquire distance data all around the medical device such as an X-ray tube head or X-ray detector in 360 degrees and if an object, for an example a moving human or movable chair or table comes from any direction, moves and comes within the guard range distance the apparatus controls the medical device such as the X-ray tube head or X-ray detector to bring it to a halt to ensure safety. However, the controller uses distance data over a limited range of angles directed around a line to the required location in order to continuously move the medical device such as the X-ray tube head or X-ray detector towards the required location that can navigate around objects and maintain a safe distance.

In an example, the controller is configured to utilize the distance data for the angular directions over the angular range either side of the direct line between the medical device and the required location at each of the plurality of locations along the route to determine a next part of the route between the initial location and the required location. The next part of the route at a location of the plurality of locations is in a direction other than the direction of the direct line between the medical device and the required location at that location if the distance data indicates that there is an object in the direction of the direct line between the medical device and the required location.

In an example, the direction of the next part of the route at the location of the plurality of locations is determined as an angular correction to the direction of the direct line between the medical device and the required location at that location calculated as an angle between a direction towards the object and a direction towards a distance mean point.

In an example, the distance mean point is determined from the distance data of the subset of the plurality of angular directions over the angular range either side of the direct line between the medical device and the required location at that location that results in a maximum distance value from the distance data for the subset of the plurality of angular directions over the angular range either side of the direct line between the medical device and the required location.

In an example, the maximum distance value is determined from a summation of distance data from at least two adjacent angular directions.

In an example, the maximum distance value is determined from the distance data of the subset of the plurality of angular directions over the angular range either side of the direct line between the medical device and the required location at that location that have been weighted, and a magnitude of a weighting value decreases as the angular direction increases away from the direct line between the medical device and the required location at that location.

Thus, the medical device such as an X-ray tube head or X-ray detector always knows the direction towards the required location as it moves, but moves around objects in a manner that it moves in a safe direction of least possibility of collision, whilst doing so in a manner that minimises the length of the route to the required location.

The weighting value therefore ensures that a route that is as close as possible to an ideal route if no object was in the way is determined, which helps to provide a shortest route to the required location.

In a second aspect, there is provided a medical device movement control system, comprising:
- an input unit;
- a medical device;
- movement system of the medical device;
- at least one distance sensor; and
- a controller.

The input unit is configured to receive a required location of the medical device. The input unit is configured to provide the required location of the medical device to the controller. The at least one distance sensor is mounted to or integrated with the medical device. The at least one distance sensor is configured to acquire distance data from the medical device for a plurality of angular directions about the medical device. The at least one distance sensor is configured to provide the distance data for the plurality of angular directions about the medical device to the controller. The controller is configured to control the movement system of the medical device to move the medical device from an initial location of the medical device to the required location along a route between the initial location and the required location that maintains a distance of at least a guard range distance between the medical device and one or more of objects comprising utilization of distance data for a subset of the plurality of angular directions acquired at a plurality of locations along the route.

In this way, a medical device such as an X-ray tube head or an X-ray detector of an X-ray system can be automatically moved continuously to a required location, and moved around objects that are in the way in moving to the final location, but automatically stop if an object, that could itself be moving such as a person, gets too close to the X-ray tube head or X-ray detector.

The medical device can for example be an X-ray tube head or X-ray detector of an X-ray system, such as an X-ray attenuation examination systems, a digital radiography system, a fluoroscopy system.

Taking the example of an X-ray system with an X-ray tube head and an X-ray detector, the controller can autonomously control movement of the X-ray tube head to a required location of the X-ray tube head, or can autonomously control movement of the X-ray detector to a required location of the X-ray detector location, where distances sensor(s) are mounted to or integrated with the X-ray tube head or X-ray detector respectively. However, distance sensors can be mounted to or integrated with the X-ray tube head and also with the X-ray detector, and a single controller can move the X-ray tube head to a required location of the X-ray tube head and move the X-ray detector to a required location of the X-ray detector, for example either side of a body part of a person.

In a third aspect, there is provided a medical device movement control method, comprising:
receiving by an input unit a required location of the medical device, and wherein at least one distance sensor is mounted to or integrated with the medical device;
providing by the input unit the required location of the medical device to a controller;
acquiring by the at least one distance sensor distance data from the medical device for a plurality of angular directions about the medical device;
providing by the at least one distance sensor the distance data for the plurality of angular directions about the medical device to the controller; and
controlling by the controller a movement system of the medical device to move the medical device from an initial location of the medical device to the required location along a route between the initial location and the required location that maintains a distance of at least a guard range distance between the medical device and one or more of objects comprising utilizing distance data for a subset of the plurality of angular directions acquired at a plurality of locations along the route.

In an aspect, there is provided a computer program element for controlling an apparatus according to the first aspect which when executed by a processor is configured to carry out the method of the third aspect.

In an aspect, there is provided a computer program element for controlling a system according to the second aspect which when executed by a processor is configured to carry out the method of the third aspect.

Thus, according to aspects, there is provided computer program elements controlling one or more of the apparatuses/systems as previously described which, if the computer program element is executed by a processor, is adapted to perform the method as previously described.

According to another aspect, there is provided computer readable media having stored the computer elements as previously described.

The computer program element can for example be a software program but can also be a Field Programmable Gated Array, a processor or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawing:
Fig. 1 shows a schematic representation of examples of uses cases of medical devices of X-ray systems;
Fig. 2 shows an example of a medical device movement control apparatus;
Fig. 3 shows an example of a medical device movement control system;
Fig. 4 shows an example of a medical device movement control method;
Fig. 5 shows an example of autonomous movement control of an X-ray tube head according to the apparatus, system and method of Figs. 2-4;
Figs. 6A-B shows examples of autonomous movement control of an X-ray tube according to the apparatus, system and method of Figs. 2-4;
Fig. 7 shows a detailed block diagram example of structural features of the apparatus, system and method of Figs. 2-4;
Fig. 8 shows an example of different types of motions associated with an exemplar X-ray tube head motion control system;
Fig. 9 shows an example of a typical X-ray room, with distances to different objects detected by the apparatus and system of Figs. 2-3 and as utilized within the method of Fig. 4 shown;
Fig. 10 shows an example of room mapping carried out by detailed embodiments of the apparatus, system and method of Figs. 2-4;
Fig. 11 shows an example of sensor placement and fields of view around an X-ray tube head as utilized by detailed embodiments of the apparatus, system and method of Figs. 2-4;
Fig. 12 shows an example of different ranges or distances as utilized by detailed embodiments of the apparatus, system and method of Figs. 2-4;
Fig. 13 shows an example of a sensor coverage area as utilized by detailed embodiments of the apparatus, system and method of Figs. 2-4;
Fig. 14 shows a top-down example of an X-ray tube head and exemplar sensor assembly locations and fields of view and shows an object in the motion path for detailed embodiments of the apparatus, system and method of Figs. 2-4;
Fig. 15 shows an example of the setting of a threshold distance for detailed embodiments of the apparatus, system and method of Figs. 2-4;
Fig. 16 shows an example of row and column averaging for a sensor for detailed embodiments of the apparatus, system and method of Figs. 2-4;
Fig. 17 shows an example of the stitching of sensor data for object location detection and course correction for detailed embodiments of the apparatus, system and method of Figs. 2-4;
Fig. 18 shows an example for a course correction angle associated with Fig. 17; and
Fig. 19 shows an example of the subsequent stitching of sensor data for object location detection and course correction following movement of the X-ray tube head associated with Figs. 17-18 for detailed embodiments of the apparatus, system and method of Figs. 2-4.

### DETAILED DESCRIPTION OF EMBODIMENTS

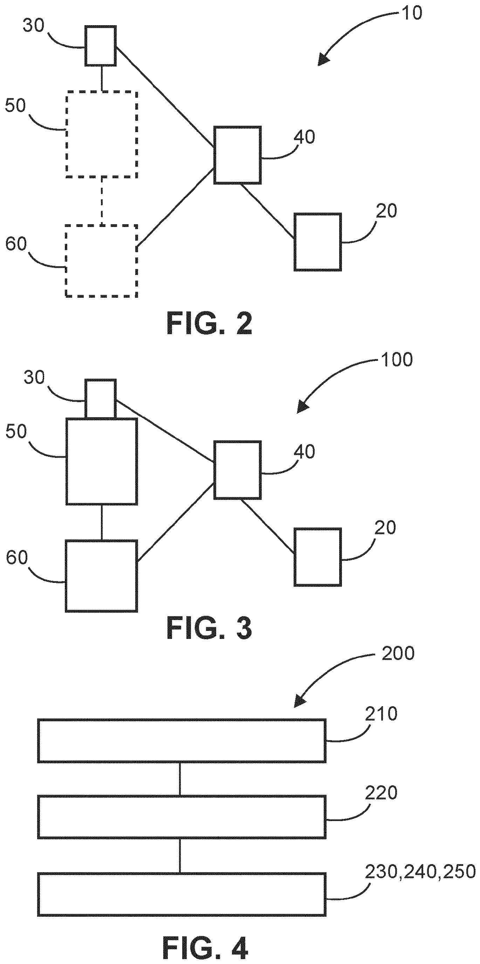

Fig. 2 shows an example of a medical device movement control apparatus 10. The apparatus 10 comprises an input unit 20, at least one distance sensor 30, and a controller 40. The input unit 20 is configured to receive a required location of the medical device 50. The input unit 20 is configured to provide the required location of the medical device 50 to the controller 40. The at least one distance sensor 30 is configured to be mounted to a medical device 50 or is configured to be integrated with the medical device 50. The at least one distance sensor 30 is configured to acquire distance data from the medical device 50 for a plurality of angular directions about the medical device 50. The at least one distance sensor 30 is configured to provide the distance data for the plurality of angular directions about the medical device 50 to the controller 40. The controller 40 is configured to control a movement system 60 of the medical device 50 to move the medical device 50 from an initial location of the medical device 50 to the required location along a route between the initial location and the required location that maintains a distance of at least a guard range distance between the medical device 50 and one or more of objects comprising utilization of distance data for a subset of the plurality of angular directions acquired at a plurality of locations along the route.

In this way, a medical device 50 such as an X-ray tube head or an X-ray detector can be automatically moved continuously to a required location, and moved around objects that are in the way in moving to the final location, but automatically stop if an object, that could itself be moving such as a person, gets too close to the X-ray tube head or X-ray detector.

The apparatus can for example be retro-fitted to an existing medical device 50 such as an X-ray tube head or X-ray detector and its movement system 60.

In an example, the medical device 50 is an X-ray tube head.

In an example, the medical device 50 is an X-ray detector.

In an example, the required location of the medical device is input into the input unit 20 by a technician based on a selected procedure.

In an example, the input unit 20 is in effect an external communication unit of the controller unit 40, configured to receive the required location from a workstation.

In an example, the input unit 20 is in effect an external communication unit of the controller unit 40, configured to receive the required location from a workstation, and wherein the required location was entered into the workstation by a technician based on a selected procedure.

Thus, the controller 40 is autonomously controlling the movement of the medical device 50 such as an X-ray tube head or X-ray detector after receiving a required location, for example from a workstation, and the workstation is not then involved further in moving the medical device 50 such as the X-ray tube head or X-ray detector.

In an example, the apparatus comprises an output unit, wherein the controller 40 is configured to utilize the output unit to report a failure or error.

In an example, the at least one distance sensor 30 comprises at least one stereo vision camera.

In an example, the at least one distance sensor 30 comprises at least one time of flight sensor.

In an example, the at least one distance sensor 30 comprises at least one lidar sensor.

In an example, the at least one distance sensor 30 comprises at least one LED burst illumination sensor.

The apparatus finds utility in attenuation X-ray examination systems, in digital radiography systems, in fluoroscopy systems for example.

According to an example, the controller 40 is configured to control the movement system 60 of the medical device 50 to bring the medical device 50 to a halt if distance data for an object indicates that it is closer to the medical device 50 than the guard range distance comprising utilization of distance data for the plurality of angular directions about the medical device 50 at one or more of the plurality of locations along the route.

In an example, the controller 40 is configured to control the movement system 60 of the medical device 50 to move the medical device 50 away from an object if distance data for the object indicates that it is closer to the medical device 50 than the guard range distance comprising utilization of distance data for the plurality of angular directions about the medical device 50 at one or more of the plurality of locations along the route.

In an example, the controller 40 is configured to control the movement system 60 of the medical device 50 to move the medical device 50 to away from an object if distance data for the object indicates that the object is approaching the guard range distance of the medical device 50 comprising utilization of distance data for the plurality of angular directions about the medical device 50 at one or more of the plurality of locations along the route.

Thus, if for example a moving object such as a person approaches the medical device 50 such as the X-ray tube head or X-ray detector the medical device 50 such as the X-ray tube head or X-ray detector can be moved away from the person to maintain a distance greater than the guard range distance before continuing on its controlled movement to the required location. However, the person or other object could be moving too fast, and enter the guard range distance and the apparatus can bring the medical device 50 such as the X-ray tube head or X-ray detector to a stop for reasons of safety. However, the apparatus can also utilize an emergency braking range that is less than the guard range distance and if the object is between the emergency braking range and the guard range distance the apparatus can determine to move the medical device 50 such as the X-ray tube head or X-ray detector away from the object, unless the object is moving too fast towards the medical device 50 such as the X-ray tube head or detector in which case the X-ray tube head or X-ray detector is stopped. However, if the object reaches the emergency brake distance from the medical device 50 such as the X-ray tube head or X-ray detector then the apparatus always stops the X-ray tube head or X-ray detector from moving.

According to an example, the distance data from the medical device 50 acquired by the at least one distance sensor 30 for the plurality of angular directions comprises distance data up to a threshold distance range that is greater than the guard range distance and less than a maximum detection range of the at least one distance sensor 30.

According to an example, the threshold is 0.5m, 1m, 1.5m, 2m, 2.5m, 3m, 3.5m.

Thus, rather than acquire actual distance data in all directions, the distance sensor 30 only passes distance information within a threshold range to the controller 40, for example only distance data for objects that are within a range of 2m of medical device 50 is provided to the controller 40, where in the direction of a wall that is 4m away the data for be set at 2m, however for objects within the threshold range of 2m actual range data, for example 1.78m would be provided to the controller. This provides for improved computational efficiency.

It is to be noted that the threshold can be less than 0.5m, between 0.5 to 1m, between 1 to 1.5m, between 1.5m to 2m, between 2 to 2.5m, between 2.5 to 3m, between 3 to 3.5m and also greater than 3m.

According to an example, the plurality of angular directions comprises a plurality of angular directions in a horizontal plane.

In an example, the plurality of angular directions in the horizontal plane covers 320 degrees, 340 degrees, 360 degrees.

In an example, the plurality of angular directions comprises a plurality of angular directions in a vertical plane.

In an example, the plurality of angular directions in the vertical plane covers 90 degrees, 120 degrees, 150 degrees, 180 degrees.

Thus, the medical device 50 such as the X-ray tube head or X-ray detector can be navigated over objects, and indeed under objects if required, as well as laterally around objects, in order to most efficiently arrive at the required location safely and quickly.

According to an example, the subset of the plurality of angular directions at each of the plurality of locations along the route comprises angular directions over an angular range either side of a direct line between the medical device 50 and the required location at each of the plurality of locations.

According to an example, the angular range either side of the direct line between the medical device 50 and the required location at each of the plurality of locations comprises an angle in the horizontal plane of 10 degrees, 15 degrees, 20 degrees, 25 degrees, 30 degrees, 35 degrees, 40 degrees, 45 degrees, 50 degrees, 55 degrees, 60 degrees.

In this manner, the at least one distance sensor 30 can acquire distance data all around the medical device 50 such as an X-ray tube head or X-ray detector in 360 degrees and if an object, for an example a moving human or movable chair or table comes from any direction, moves and comes within the guard range distance the apparatus controls the medical device 50 such as the X-ray tube head or X-ray detector to bring it to a halt to ensure safety. However, the controller 40 uses distance data over a limited range of angles directed around a line to the required location in order to continuously move the medical device 50 such as the X-ray tube head or X-ray detector towards the required location that can navigate around objects and maintain a safe distance.

In an example, the angular range either side of the direct line between the medical device and the required location at each of the plurality of locations comprises an angle in the vertical plane of 10 degrees, 15 degrees, 20 degrees, 25 degrees, 30 degrees, 35 degrees, 40 degrees, 45 degrees, 50 degrees, 55 degrees, 60 degrees.

According to an example, the controller 40 is configured to utilize the distance data for the angular directions over the angular range either side of the direct line between the medical device 50 and the required location at each of the plurality of locations along the route to determine a next part of the route between the initial location and the required location. The next part of the route at a location of the plurality of locations is in a direction other than the direction of the direct line between the medical device 50 and the required location at that location if the distance data indicates that there is an object in the direction of the direct line between the medical device 50 and the required location.

According to an example, the direction of the next part of the route at the location of the plurality of locations is determined as an angular correction to the direction of the direct line between the medical device 50 and the required location at that location calculated as an angle between a direction towards the object and a direction towards a distance mean point.

In an example, the distance mean point is a maximum geometric distance mean point.

In an example, the distance mean point is a weighted mean distance mean point.

The distance mean point can be determined by techniques such as a windowing technique.

According to an example, the distance mean point is determined from the distance data of the subset of the plurality of angular directions over the angular range either side of the direct line between the medical device 50 and the required location at that location that results in a maximum distance value from the distance data for the subset of the plurality of angular directions over the angular range either side of the direct line between the medical device and the required location.

According to an example, the maximum distance value is determined from a summation of distance data from at least two adjacent angular directions.

According to an example, the maximum distance value is determined from the distance data of the subset of the plurality of angular directions over the angular range either side of the direct line between the medical device 50 and the required location at that location that have been weighted, and wherein a magnitude of a weighting value decreases as the angular direction increases away from the direct line between the medical device and the required location at that location.

Thus, the medical device 50 such as an X-ray tube head or X-ray detector always knows the direction towards the required location as it moves, but moves around objects in a manner that it moves in a safe direction of least possibility of collision, whilst doing so in a manner that minimizes the length of the route to the required location.

The weighting value therefore ensures that a route that is as close as possible to an ideal route if no object was in the way is determined, which helps to provide a shortest route to the required location.

Fig. 3 shows an example of a medical device movement control system 100. The system 100 comprises an input unit 20, a medical device 50, a movement system 60 of the medical device 50, at least one distance sensor 30, and a controller 40. The input unit 20 is configured to receive a required location of the medical device 50. The input unit 20 is configured to provide the required location of the medical device 50 to the controller 40. The at least one distance sensor 30 is mounted to or integrated with the medical device 50. The at least one distance sensor 30 is configured to acquire distance data from the medical device 50 for a plurality of angular directions about the medical device 50. The at least one distance sensor 30 is configured to provide the distance data for the plurality of angular directions about the medical device 50 to the controller 40. The controller 40 is configured to control the movement system 60 of the medical device 50 to move the medical device 50 from an initial location of the medical device 50 to the required location along a route between the initial location and the required location that maintains a distance of at least a guard range distance between the medical device 50 and one or more of objects comprising utilization of distance data for a subset of the plurality of angular directions acquired at a plurality of locations along the route.

In an example, the medical device 50 is an X-ray tube head.

In an example, the medical device 50 is an X-ray detector.

In an example, the system is an attenuation X-ray examination system.

In an example, the system is a digital radiography system.

In an example, the system is a fluoroscopy system

In an example, the controller 40 is configured to control the movement system 60 of the medical device 50 to bring the medical device 50 to a halt if distance data for an object indicates that it is closer to the medical device 50 than the guard range distance comprising utilization of distance data for the plurality of angular directions about the medical device 50 at one or more of the plurality of locations along the route.

In an example, the controller 40 is configured to control the movement system 60 of the medical device 50 to move the medical device 50 to away from an object if distance data for the object indicates that it is closer to the medical device 50 than the guard range distance comprising utilization of distance data for the plurality of angular directions about the medical device 50 at one or more of the plurality of locations along the route.

In an example, the controller 40 is configured to control the movement system 60 of the medical device 50 to away from an object if distance data for the object indicates that the object is approaching the guard range distance of the medical device 50 comprising utilization of distance data for the plurality of angular directions about the medical device 50 at one or more of the plurality of locations along the route.

In an example, the distance data from the medical device 50 acquired by the at least one distance sensor 30 for the plurality of angular directions comprises distance data up to a threshold distance range that is greater than the guard range distance and less than a maximum detection range of the at least one distance sensor 30.

In an example, the threshold is 0.5m, 1m, 1.5m, 2m, 2.5m, 3m, 3.5m.

In an example, the plurality of angular directions comprises 360 degrees in a horizontal plane.

In an example, the plurality of angular directions comprises a plurality of angular directions in a horizontal plane.

In an example, the plurality of angular directions in the horizontal plane covers 320 degrees, 340 degrees, 360 degrees.

In an example, the plurality of angular directions comprises a plurality of angular directions in a vertical plane.

In an example, the plurality of angular directions in the vertical plane covers 90 degrees, 120 degrees, 150 degrees, 180 degrees.

Thus, the medical device 50 such as an X-ray tube head or X-ray detector can be navigated over objects, and indeed under objects if required, as well as laterally around objects, in order to most efficiently arrive at the required location safely and quickly.

In an example, the subset of the plurality of angular directions at each of the plurality of locations along the route comprises angular directions over an angular range either side of a direct line between the medical device and the required location at each of the plurality of locations.

In an example, the angular range either side of the direct line between the medical device and the required location at each of the plurality of locations comprises an angle in the horizontal plane of 10 degrees, 15 degrees, 20 degrees, 25 degrees, 30 degrees, 35 degrees, 40 degrees, 45 degrees, 50 degrees, 55 degrees, 60 degrees.

In an example, the angular range either side of the direct line between the medical device and the required location at each of the plurality of locations comprises an angle in the vertical plane of 10 degrees, 15 degrees, 20 degrees, 25 degrees, 30 degrees, 35 degrees, 40 degrees, 45 degrees, 50 degrees, 55 degrees, 60 degrees.

In an example, the controller 40 is configured to utilize the distance data for the angular directions over the angular range either side of the direct line between the medical device 50 and the required location at each of the plurality of locations along the route to determine a next part of the route between the initial location and the required location. The next part of the route at a location of the plurality of locations is in a direction other than the direction of the direct line between the medical device 50 and the required location at that location if the distance data indicates that there is an object in the direction of the direct line between the medical device 50 and the required location.

In an example, the direction of the next part of the route at the location of the plurality of locations is determined as an angular correction to the direction of the direct line between the medical device 50 and the required location at that location calculated as an angle between a direction towards the object and a direction towards a distance mean point.

In an example, the distance mean point is a maximum geometric distance mean point.

In an example, the distance mean point is a weighted mean distance mean point.

The distance mean point can be determined by techniques such as a windowing technique.

In an example, the distance mean point is determined from the distance data of the subset of the plurality of angular directions over the angular range either side of the direct line between the medical device 50 and the required location at that location that results in a maximum distance value from the distance data for the subset of the plurality of angular directions over the angular range either side of the direct line between the medical device 50 and the required location.

In an example, the maximum distance value is determined from a summation of distance data from at least two adjacent angular directions.

In an example, the maximum distance value is determined from the distance data of the subset of the plurality of angular directions over the angular range either side of the direct line between the medical device 50 and the required location at that location that have been weighted, and wherein a magnitude of a weighting value decreases as the angular direction increases away from the direct line between the medical device 50 and the required location at that location.

Fig. 4 shows an example of a medical device movement control method 200. The method 200 comprises:
receiving 210 by an input unit 20 a required location of the medical device 50, and wherein at least one distance sensor 30 is mounted to or integrated with the medical device 50;
providing 220 by the input unit 20 the required location of the medical device 50 to a controller 40;
acquiring 230 by the at least one distance sensor 30 distance data from the medical device 50 for a plurality of angular directions about the medical device 50;
providing 240 by the at least one distance sensor 30 the distance data for the plurality of angular directions about the medical device 50 to the controller 40; and
controlling 250 by the controller 40 a movement system 60 of the medical device to move the medical device 50 from an initial location of the medical device 50 to the required location along a route between the initial location and the required location that maintains a distance of at least a guard range distance between the medical device 50 and one or more of objects comprising utilizing distance data for a subset of the plurality of angular directions acquired at a plurality of locations along the route.

In an example, the medical device 50 is an X-ray tube head.

In an example, the medical device 50 is an X-ray detector.

In an example, the method comprises controlling by the controller 40 the movement system 60 of the medical device 50 to bring the medical device 50 to a halt if distance data for an object indicates that it is closer to the medical device 50 than the guard range distance comprising utilizing distance data for the plurality of angular directions about the medical device 50 at one or more of the plurality of locations along the route.

In an example, the method comprises controlling by the controller 40 the movement system 60 of the medical device 50 to move the medical device 50 to away from an object if distance data for the object indicates that it is closer to the medical device 50 than the guard range distance comprising utilizing distance data for the plurality of angular directions about the medical device 50 at one or more of the plurality of locations along the route.

In an example, the method comprises controlling by the controller 40 the movement system 60 of the medical device 50 to move the medical device 50 to away from an object if distance data for the object indicates that the object is approaching the guard range distance of the medical device 50 comprising utilization of distance data for the plurality of angular directions about the medical device 50 at one or more of the plurality of locations along the route.

In an example, the distance data from the medical device 50 acquired by the at least one distance sensor 30 for the plurality of angular directions comprises distance data up to a threshold distance range that is greater than the guard range distance and less than a maximum detection range of the at least one distance sensor 30.

In an example, the threshold is 0.5m, 1m, 1.5m, 2m, 2.5m, 3m, 3.5m.

In an example, the plurality of angular directions comprises a plurality of angular directions in a horizontal plane.

In an example, the plurality of angular directions in the horizontal plane covers 320 degrees, 340 degrees, 360 degrees.

In an example, the plurality of angular directions comprises a plurality of angular directions in a vertical plane.

In an example, the plurality of angular directions in the vertical plane covers 90 degrees, 120 degrees, 150 degrees, 180 degrees.

In an example, the subset of the plurality of angular directions at each of the plurality of locations along the route comprises angular directions over an angular range either side of a direct line between the medical device 50 and the required location at each of the plurality of locations.

In an example, the angular range either side of the direct line between the medical device 50 and the required location at each of the plurality of locations comprises an angle in the horizontal plane of 10 degrees, 15 degrees, 20 degrees, 25 degrees, 30 degrees, 35 degrees, 40 degrees, 45 degrees, 50 degrees, 55 degrees, 60 degrees.

In an example, the angular range either side of the direct line between the medical device 50 and the required location at each of the plurality of locations comprises an angle in the vertical plane of 10 degrees, 15 degrees, 20 degrees, 25 degrees, 30 degrees, 35 degrees, 40 degrees, 45 degrees, 50 degrees, 55 degrees, 60 degrees.

In an example, the method comprises utilizing by the controller 40 the distance data for the angular directions over the angular range either side of the direct line between the medical device 50 and the required location at each of the plurality of locations along the route to determine a next part of the route between the initial location and the required location. The next part of the route at a location of the plurality of locations is in a direction other than the direction of the direct line between the medical device 50 and the required location at that location if the distance data indicates that there is an object in the direction of the direct line between the medical device 50 and the required location.

In an example, the direction of the next part of the route at the location of the plurality of locations is determined as an angular correction to the direction of the direct line between the medical device 50 and the required location at that location calculated as an angle between a direction towards the object and a direction towards a distance mean point.

In an example, the distance mean point is a maximum geometric distance mean point.

In an example, the distance mean point is a weighted mean distance mean point.

The distance mean point can be determined by techniques such as a windowing technique.

In an example, the distance mean point is determined from the distance data of the subset of the plurality of angular directions over the angular range either side of the direct line between the medical device 50 and the required location at that location that results in a maximum distance value from the distance data for the subset of the plurality of angular directions over the angular range either side of the direct line between the medical device 50 and the required location.

In an example, the maximum distance value is determined from a summation of distance data from at least two adjacent angular directions.

In an example, the maximum distance value is determined from the distance data of the subset of the plurality of angular directions over the angular range either side of the direct line between the medical device 50 and the required location at that location that have been weighted, and wherein a magnitude of a weighting value decreases as the angular direction increases away from the direct line between the medical device and the required location at that location.

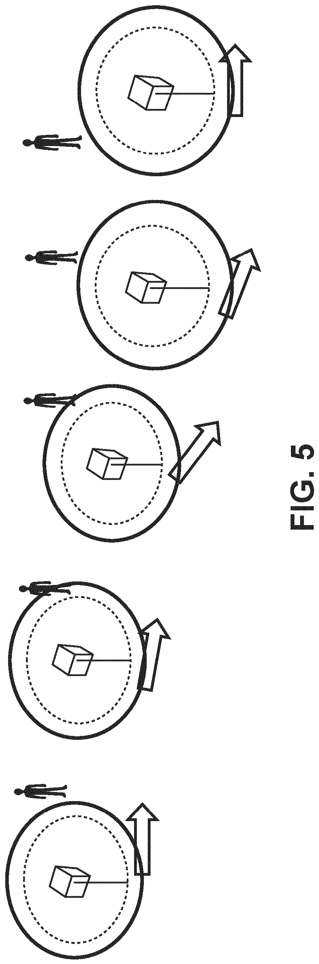

The medical device movement control apparatus, the medical device movement control system, and the medical device movement control method are now described in specific detail, where reference is made to Figs. 5-19. The following description relates for reasons of simplicity of description generally to the movement of a medical device 50 in the form of an X-ray tube head. However, the new development finds utility to X-ray tube heads, X-ray detectors and other medical devices, and can be used in attenuation X-ray examination systems, in digital radiography systems, in fluoroscopy systems for example.

It was realised that problems associated with existing X-ray tube head movement control systems, that bring the X-ray tube head to head after a collision or prior to a collision, could be addressed by providing a system that is continuously aware of the surroundings and objects in the vicinity of the X-ray tube head, and that detects the possibility of collision and then recalculates another safer route to avoid the collision and that enables the motion of the X-ray tube head to be continued to the desired or required location. Advanced collision detection then eliminates the possibility of collision of the X-ray tube head with objects from a longer range, and the system can slow down the X-ray tube head safely, thereby reducing long term wear and tear, before continuing along an alternative route, or if necessary, bringing the system to a complete halt in emergencies. Autonomous trajectory determination enables the X-ray tube head to be automatically moved from a start point to and endpoint or required location. The system continues motion, even during a possibility of collisions with objects, by automatically finding an on-the-go alternate path avoiding collisions with objects, and this operator intervention is fully eliminated for the automated movement of the X-ray tube head after they indicate a required location for the X-ray tube head that can be that as required for a specific procedure. This then saves precious "Operator" time and reduces 'Operator fatigue' significantly in the operation/ X-ray room. It was also realized that X-ray detectors as well as X-ray tube heads could be controlled to be automatically and safely brought to their correct locations for an examination or scan.

Figs. 5-6A-B show examples of collision avoidance and autonomous movement control of an X-ray tube head as it continuously moves towards a required location, whilst navigating around objects in the way.

Advanced sensing enables the system to continuously sense the configurable distances around the X-ray tube head up to distances of a few meters, which allows more margin in terms of latency to the motion controller. Long sensing distance enables objects to be detected from longer distances, and enables the early navigating around objects and if necessary, enables slowing down the velocity of the X-ray tube head and enables smoother stoppage rather than applying emergency brakes that would result in abrupt stoppage.

The sensors 30 are mounted on the X-ray tube head and utilize time of flight (TOF) sensing, and can be lidar sensors or LED illumination sensors or camera and/or depth sensing based systems, where the detector for each sensor 30 can be for example a single detector pixel for a scanning lidar sensor system or a 2D detector array for a burst illumination lidar sensor system or burst illumination LED system. The sensor system 30 can sense distances from 2-3 cm up to 2-3 meters with a large Field of view of 60 degrees for each, thereby enabling tracking of significant movements in a particular area around the X-ray tube head and where a number of sensors can provide for 360 degree sensing around the X-ray tube head. By using a TOF sensing technique the sensing range can be adjusted by only analyzing reflected light that returns within a certain time window of the light emission. As the time window increases, the sensing range threshold increases. Thus, a time window of approximately 18ns means that objects further than 3m away are not sensed, and in this way by adjusting the threshold the "noise" or amount of data to be analyzed can be reduced. Thus, a variable threshold distance can be utilized, and to reduce noise once an object has been detected the variable threshold can be set as the distance to a closest part of the object, and therefore varies as the X-ray tube head is moved.

Multizone sensing is provided, where the sensing mechanism divides the sensing field of view into multiple finer zones rather than one single area. Each zone can be configured with different ranging thresholds enabling a uniform spherical sensing with respect to the X-ray tube head, thereby giving a precise range sensing threshold which can avoid unwanted sensing of the object out of the sensing range

Motion detection is utilized as part of the advanced anti-collision system in order to detect objects that are moving (e.g. humans). The system can efficiently distinguish between stationary and moving objects by analyzing adjacent sensing cycles to determine if an object is at a fixed location consistent with the movement of the X-ray tube head or if the object has moved. Thus, the system in effect performs histogram based sensing to provide the motion controller information about objects to create alternate trajectory and also take a real time decision to avoid collision. For example, if a human is walking slowly towards the X-ray tube head from any direction, the X-ray tube head can be controlled to navigate away from the human before continuing with the movement towards the required location. However, if the person is very moving towards the X-ray tube head the x-ray tube head can be controlled to come to a halt before there is a collision. Once the human has moved away from a guard range distance the X-ray tube head can then be controlled to continue its movement towards the required location.

Thus, the X-ray tube head can continue with its motion even during a possibility of collisions by automatically finding an on-the-go alternate path avoiding collision. This eliminates operator intervention completely and also avoids operator fatigue, and a complete one touch automated motion system can be provided, for example where an operator can simply press a procedure button in a UI workflow on a workstation and the system then automatically performs the movement of the X-ray tube head to carry out this procedure without any operator intervention and without the workstation doing anything further. This is because the system provides a mechanism with which the system can find its own alternate route for the X-ray tube head if objects are detected in the vicinity of the X-ray tube head, where the system can not only avoid collision but also can continue uninterrupted movement from start to end coordinates (the required location).

Thus, referring to Fig. 5, the first image represents a desired direction movement of the X-ray tube head (within the two circles) towards a required location. The inner circle indicates a guard range distance around the X-ray tube head that the system will move the X-ray tube head in order that objects are maintained at a distance of at least the guard range distance from the X-ray tube head, unless as discussed above if a fast moving object for example approaches the X-ray tube head and the system brings the X-ray tube head to a stop in order that there is a safe situation as the object gets closer to the X-ray tube head than the guard range distance. The second circle around the X-ray tube head is the variable threshold distance, as discussed above that is the distance around the X-ray tube head where distance data are analyzed for objects. The second image shows a change in direction of the X-ray tube as it approaches the person. The third image shows that collision has been avoided, and where the X-ray tube head movement has continued. The fourth image shows that the system is calibrating a new direction to move the X-ray tube head to the required location. The fifth image then indicates that the motion of the X-ray tube head is continuing towards the required location.

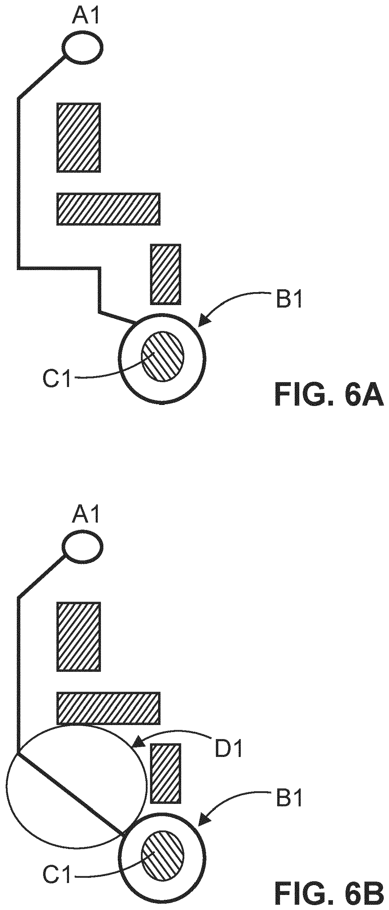

The collision avoidance and automatic movement system is shown in further detail in Figs. 6A-B. In Fig. 6A-B the required location, or destination, to which the X-ray tube head is to be automatically moved is indicated as "A1". The X-ray tube head is indicated as "C1". The guard range distance is indicated as "B1". The threshold distance range is indicated as "D1". As part of a coordinate based motion system a motion controller, that can be in the X-ray tube head, receives information about end coordinates (motion coordinates of a required location) from a workstation. The start position can already be known and can be the location where the X-ray tube head is currently located. However, the workstation can provide the controller with a start location that the X-ray tube head will move to and then move from that location to the required location. However, in this situation the start location is in effect a required location and the system operates in the collision avoidance and automatic movement functionalities when moving to the start location and then also when moving from the start location to the required location.

Continuing with Figs. 6A-B, as shown in the Fig. 6A, the system can utilize only a guard range distance B 1 in avoiding collision and moving towards the required location, but this can lead to a jerky route that is not the shortest and can lead to constant adjustment of velocity and acceleration. However, in Fig. 6B a variable threshold distance D1 can be utilized in addition to the guard range distance. The system sets a variable threshold distance to obtain information about the free space around the X-ray tube head and the next obstacle on the path. This approach helps in terms of user experience by eliminating creep movements/system entrapments and saves time, and can actually result in the shortest path. As well as a shorter path higher accelerations and velocities can be achieved with much smoother motor movements.

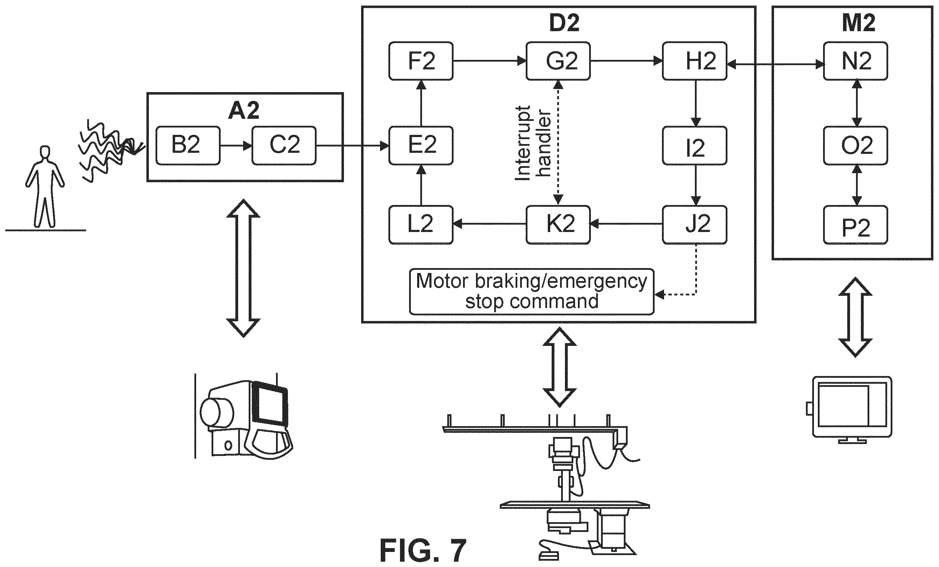

Fig. 7 shows a block diagram of a detailed medical device movement control apparatus/system/method, and depicts an overall flow of the apparatus/system/method catering for both collision avoidance and trajectory control. In Fig. 7 Sensor front end is indicated as "A2" and relates to the box containing "B2 and "C2". The sensors 30 of the medical device 50 are indicated by "B2", and the sensor raw data acquired by the sensors 30, B2 is indicated as "C2", The functionality of the motion controller 40 is indicated as "D2", Position set threshold computation is indicated as "E2", Zonal relative velocity calculation is indicated as "F2", Trajectory control logic is indicated as "G2", New trajectory setpoint generator is indicated as "H2", Guard range check is indicated as "12", Collision avoidance logic is indicated as "J2", To motor drives is indicated as "K2", Motor position feedback is indicated as "L2", Operator interface is indicated as "M2", Room layout and coordinate data is indicated as "N2", Examination information is indicated as "O2", and Workspot (operator's console) is indicated as "P2". An operator uses a workstation P2 from which a required location for the medical device is set, that can take into account the layout of the room N2, and information relating to the examination to be carried out O2. The medical device 50 has sensors 30, B2, that acquire raw data C2, and a motion controller 40, D2 utilizes this data in moving the medical device 50 to the required location. In effect a feedback loop type mechanism is utilized, where the raw data C2 is used to set position thresholds E2, following which velocity calculations F2 are made. Control logic G2 is used in generating a new trajectory H2 that involves a guard range check 12, and the utilization of collision avoidance logic J2, and if necessary an emergency stop is made. Instructions K2 are sent to a movement control system 60 to move the medical device 50, and position feedback L2 is utilized with newly acquired sensor data C2 to continue to loop round this process to move the medical device 50 to the required location.

The medical device movement control apparatus/system/method is compatible with any type of range/depth/vision sensor 30. The new technology is a combination of algorithm and framework, and can provide autonomous motion irrespective of the type of sensor 30 used (Camera, LiDAR, mmWAVE, Ultrasound or any type of combinational arrays). This is possible as the system makes use of real time sensor data to extract the spacing of objects from the X-ray tube head in 3D space.

Continuing with Fig. 7 the Sensor Front End A2 that has sensors B2, 30 of a medical device 50 and that acquires sensor data C2 is an independent entity with independent functionality with respect to the rest of the algorithm. Regarding the medical device 50 (such as the X-ray tube head or X-ray detector), with respect to collision avoidance and autonomous movement, there are a number of motions to control at any given point of time, where the sensor data C2 acquired by the sensors 30, B2 is utilized by the controller 40, D2, as shown in Fig. 7 and as discussed above, to move the medical device 50 to the required location whilst moving around objects that are in the way. In one example, lateral and longitudinal motions, and indeed vertical motions, are the movements for Autonomous trajectory (e.g. movement of X-ray tube head/X-ray detector from X1, Y1 position to X2, Y2 position (and Z1 to Z2 if necessary)) and control of velocity, while all the motions can also be controlled for collision avoidance. Thus, for example if there is any object right below the X-ray tube head/detector, the height of the X-ray tube head/detector can be changed first along with Alpha/Beta motion to avoid collision and then Lateral and Longitudinal motion can take place for the autonomous trajectory motion.

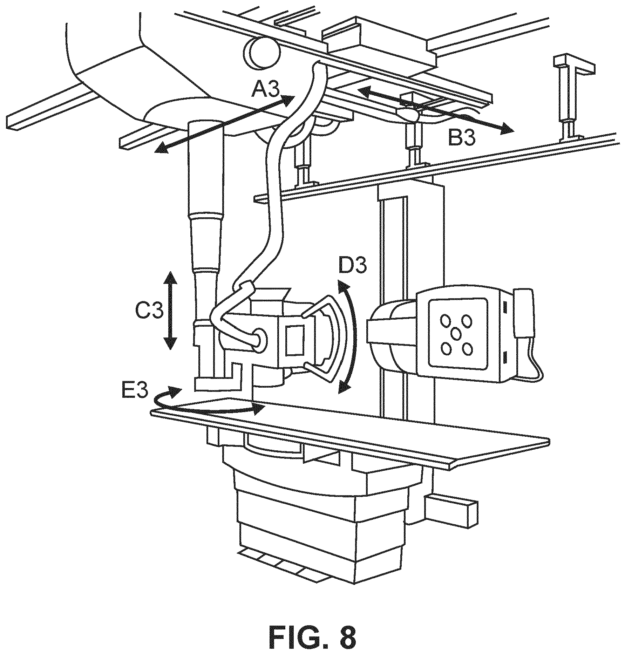

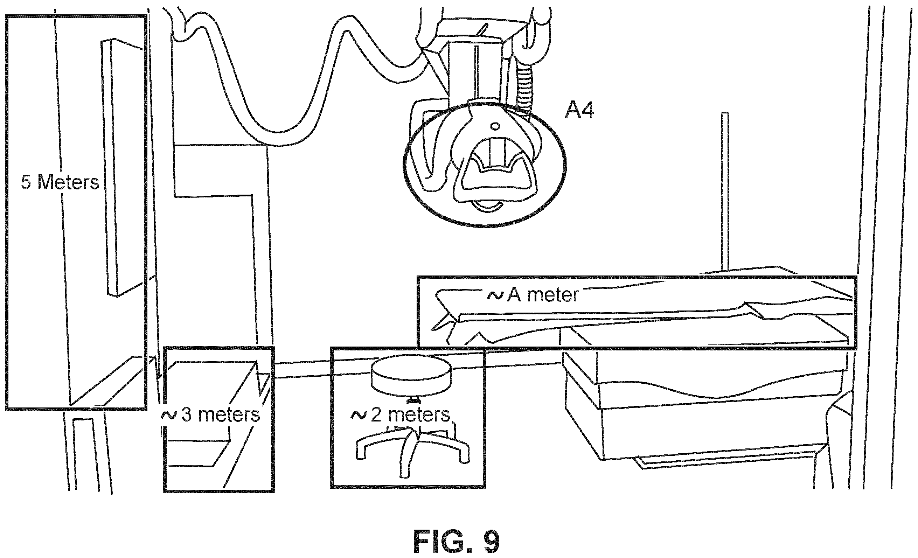

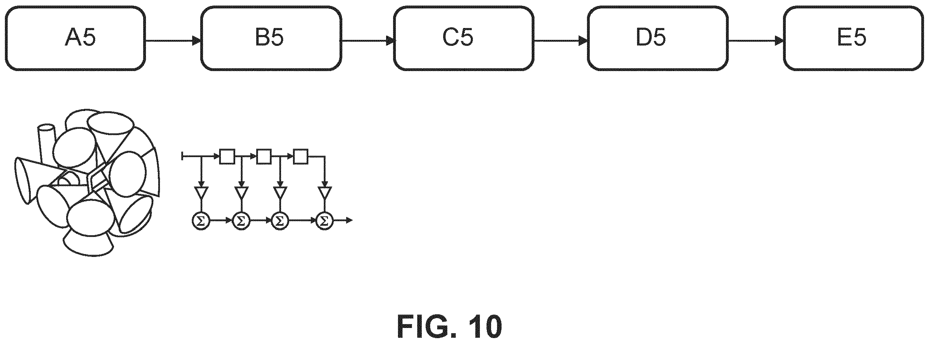

The new system provides for variable distance sensing. As shown in the Figs 8-9, a typical X-ray room has many fixed and movable parts apart from the human presence. In Fig. 8 long is indicated by "A3", lat is indicated by "B3", Height is indicated by "C3" (lat, long, and height can be considered to be equivalent to x, y, and z), tilt alpha is indicated by "D3", and arm swing is indicated by "E3", where these are representative movements that the medical device 50 in terms of an X-ray tube head moves from a start position or location to a required position or location. In Fig. 9 sensor assembly is indicated by "A4", and the system can sense the range to different objects in the room as shown. Thus, variable range sensing can locate the distance to objects and if necessary create an object map of an X-ray room (as shown in Fig. 10) but also to change the real time navigation path as shown as shown in Figs. 5-6. In Fig. 10 a room mapping routine is shown, where multi sensor raw data is indicated as "A5", digital filters is indicated by "B5", room floor and object mapping (coordinates) is indicated by "C5", key coordinates with respect to distance table is indicated by "D5", and motion controller 40 is indicated by "E5" - the motion controller 40 is also referred to as D2 in Fig. 7.

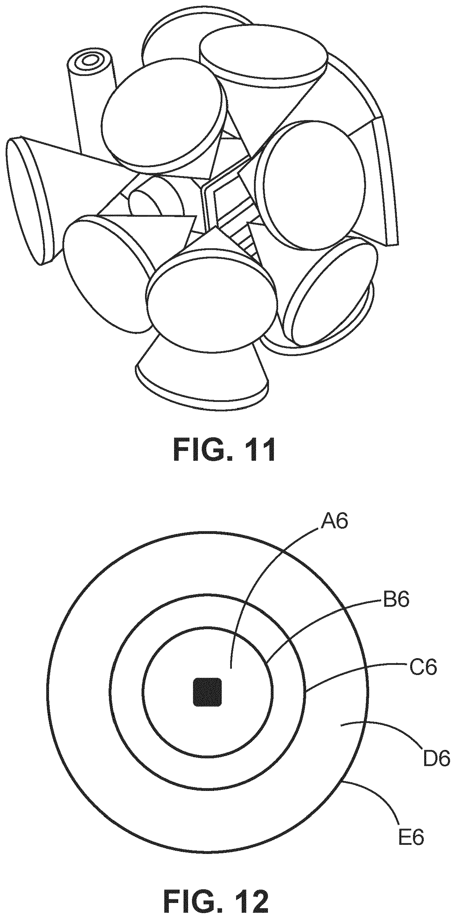

Fig. 11 shows a detailed example of sensor placement around an X-ray tube head/X-ray detector and showing the fields of view of individual sensors, where the field of view of all the sensors can be nearly 360 degrees.

Thus, with respect to the above discussion, features presented in Fig. 7 can then be discussed in further detail.

### Sensor Front End A2

Thus, a sensor front end A2 has an array of sensors B2, 30 looking in various directions creating a sphere around the medical device 50 (X-ray tube head/X-ray detector) that acquire raw data C2. The radius of the sphere can be software configurable to create a body guard range (guard range distance) and threshold range (threshold distance), by interrogating data within different time windows as discussed previously. The sensor front end A2 can then transfer the raw sensing data C2 to the motion controller D2, 40.

### Motion Controller D2

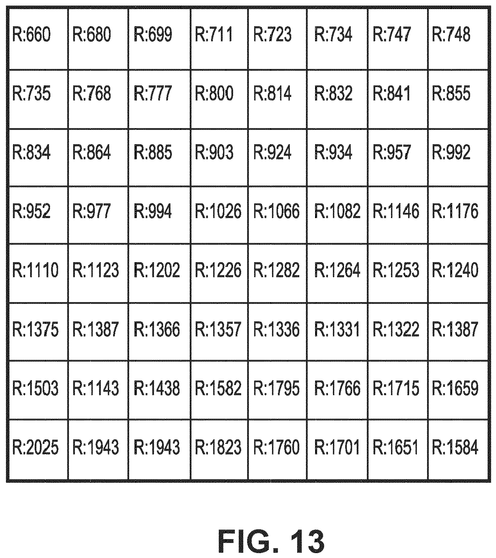

The motion controller D2, 40 is a closed loop control system receiving the sensor raw data C2, position feedback for motors and coordinate based data inputs as its important inputs. The controller D2, 40 works completely in real time for collision avoidance, constantly sensing the sensor raw data against the set threshold. The moment any deviation inward to the set threshold limits is detected on any of the sensor's individual pixels, the motion controller D2, 40 takes immediate action to avoid the collision and either stop/find another route based on the configuration via a movement system 60. The sensor front end A2 thus transfers the raw sensing data C2 from sensors B2, 30 to the motion controller D2, 40. Each sensor in itself sees the field of view as a group of pixels (for ex: 8 by 8 = 64 pixel matrix shown in Fig. 13). Each matrix denotes a portion of the spatial field across the sensing surface of a sensor up to a certain distance of a few meters. This creates a real time array of distances, which is the raw data for implementation of guard range and avoidance logic. From the block level diagram, there are other elements of the design, such as trajectory control logic G2, relative velocity calculator F2, set-threshold computation logics E2 along with other elements, that work together towards automatic trajectory control.

### Position Set Threshold Computation E2

This block E2 is responsible for setting the threshold for the body guard range (collision avoidance) and variable threshold for sensing distance.

### Zonal Relative Velocity Computation F2

This block F2 makes use of distance sensing arrays on the sensors B2, 30. These arrays are available on sensors such as Cameras, Time of flights, Ultrasound sensors etc. The Array inside the sensor divides the field into smaller areas (Zones) to increase the sensing resolution and thereby create a better map of objects and their distances from the medical device 50 (X-ray tube head/X-ray detector). The measured distances are used to determine the best possible direction and speed/acceleration to move towards the required location.

### Trajectory control logic G2

Based on the room map, present position, present location of the medical device 50 (X-ray tube/X-ray detector) head in 3D space, this block G2 takes the decision of what is the best next direction the X-ray tube head/X-ray detector can move into.

### New Trajectory Setpoint Generator H2

For example when a technician sets the procedure (Orto - Knee vs Spine for ex), a trajectory for the medical device 50 (X-ray tube head/X-ray detector) is generated. Based on the direction from the previous block and of the next trajectory a real time set of next motion coordinates are continuously generated by this block H2. The next motion coordinates are used to provide jerk free/ smooth motion and also should be free from movements leading to dead ends/blocks.

### Guard Range Check 12 and Collision Avoidance Logic J2

These two blocks 12 and J2 work together to keep checking if any object is sensed inside the body guard range setup by the system. This block is executed repetitively to minimize the risk of collision.

Fig. 12 shows the different ranges utilized within a detailed embodiment of the apparatus/system/method. In Fig. 12 the medical device 50, such as an X-ray tube head/X-ray detector, is indicated as "A6", Emergency braking range is indicated by "B6", guard range is indicated as "C6", Variable distance thresholding zone is indicated as "D6", and Maximum sensing radius is indicated as "E6".

Continuing with Fig. 12, with respect to the medical device 50, A6 (e.g. X-ray tube head) this is a part of an X-ray system where an X-ray generator is present along with other key system parts such as collimator, X-ray tube, user interface, handles, and motorization system 60. It is to be noted that a plurality of time of flight sensors 30 are discussed that can be mounted around the X-ray tube head. Pseudo three dimensional boundaries can be created by stitching together data from the various sensors mounting around the X-ray tube head that cover all of its sides.

The emergency Braking distance (range) is the distance (range) within which if the presence of any object is detected, the system takes immediate action to apply brakes and come to a standstill with least latency. In this range, there is a strong possibility of collision.

Guard range (also called body guard range) is a minimum fixed distance within which if any object enters, the system assumes a possibility of collision and comes to a standstill with a defined deacceleration. This distance can be software configurable, but once configured can remain fixed during the system operation.

Threshold distance is a maximum distance within which the system assumes free space with minimum collision risk. The higher the threshold distance, the system can find the best and fastest trajectory to the destination but the system can also experience several noise sources and will be required to reduce threshold distance frequently. A lower threshold distance can however lead to inferior motion trajectories motions but offers better noise performance- see for example the two examples of Fig. 6.

Variable distance thresholding zone is a zone in which the algorithm takes a decision on direction and speed of motion towards the final destination (required location) based on the presence of objects in this region.

Maximum sensing distance relates to a maximum sensing range used for a sensor 30 that can have a range up to a few meters. The higher the sensing range will mean more coverage, but it will also mean more noise as sensors will pick up more objects/movements which are unwanted (hence termed as noise). As a "Sweet spot" of performance vs coverage, the sensing distance of sensors can be appropriately selected so that the noise performance is not too large, whilst at the same time the system accuracy is not compromised. It is to be noted that the depth/sensing range of sensors needs to be equal or greater than this sensing distance.

Fig. 13 shows a sensor coverage area distributed into smaller pixels, where values in each pixel indicates a distance from the medical device 50 (X-ray tube head/X-ray detector). As discussed above, the sensors 30 are TOF (Time of flight) based sensors, which can be replaced by any other type of depth sensing technique such as ultrasound, camera, IR array, LiDAR etc. provided they divide their sensing area in form of pixels such as an 8x8 pixel array shown in Fig. 13. These sensor pixels are smaller areas of larger area which provide finer details and are used for data processing, and where each sensor pixel can have its own threshold range such that a sphere of detection range can be generated around the X-ray tube head/X-ray detector.

Sensors 30 are then arranged on the medical device 50 such as the X-ray tube head/X-ray detector to provide 360-degree coverage around the X-ray tube head/X-ray detector.

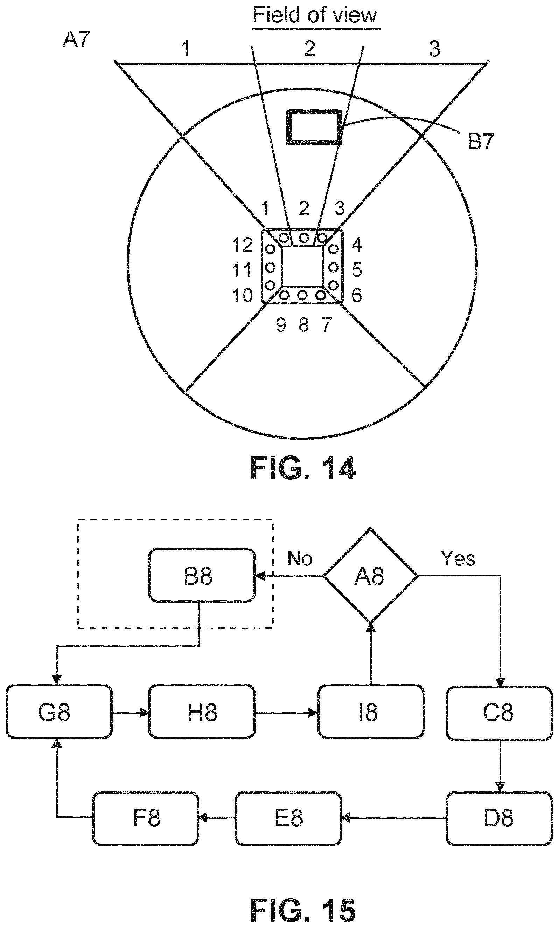

Fig. 14 then shows a projection of three of 12 sensors 30 mounted on medical device 50 (X-ray tube head/X-ray detector). Each sensor 30 has its own field of view. A wide FOV is obtained by stitching the sensor data by sensor front end acquisition logic. Also as discussed above, rather than this flat end of detection, this can be curved to form a sphere around the X-ray tube head/X-ray detector. In Fig. 14 sensor is indicated by "A7", and object in motion path is indicated by "B7". Fig. 14 represents one example of sensor placements, where 12 sensors are utilized with 3 on each of 4 directions. However, a single scanning sensor can scan 360 degrees, 2 sensors can each scan 180 degrees, 4 sensors can be used, one for each directions, 8 sensors can be used, 2 for each direction, and indeed more than 12 sensors can be utilized.

Fig. 15 then details how the threshold distance can be set. In Fig 15 Is sensed distance >= guard range distance is indicated by "A8", Abort, raise emergency flag is indicated by "B8", data stitching is indicated by "C8", set new distance threshold is indicated by "D8", compute acceleration vector (direction and value) is indicated by "E8", set new direction is indicated by "F8", Receive sensor raw data is indicated by "G8", Digital FIR (Finite Impulse Response) filters is indicated by "H8", Moving windowing average (Geometric mean) of rows and columns is indicated by "18".

Continuing with Fig. 15 the medical device motion controller 40 receives end destination coordinates from work spot. The system scans for surrounding objects (if any object is present in the guard range distance the system raises a warning). However, the system has the ability to try to navigate away from any object present inside its guard range. This is achieved by using sensors 30 to scan the area 360 degrees, and if there is no block seen in any other direct, the motion controller 40 will navigate away from the object to take it out of the guard range distance. Once the end destination (required location) is with system and no object is detected in the guard range distance, the motion controller 40 starts to move the medical device 50 (X-ray tube head/X-ray detector) using a movement system 60, with an initial distance threshold equal to the guard range distance. This distance threshold can change over the course of motion based on surrounding objects and the direction of the end destination (required location). The loop shown in Fig 15 is executed continuously in real time until the X-ray tube head/X-ray detector reaches the required location.

With respect to Fig. 15, the following discussion provides more details on specific elements.

The FIR filter is a 2nd /3rd order FIR filter, applied on each pixel of consecutive frames of a particular sensor to obtain lower noise data. This is used to smooth out any false readings (arising mainly due to reflections etc.). The specific order of the filter used can be selected with respect to a tradeoff with filter latency.

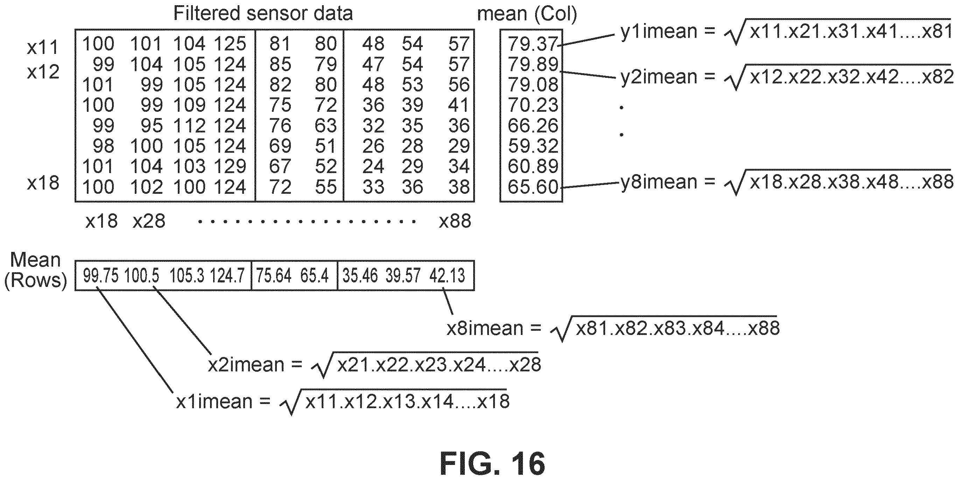

Fig. 16 shows row and column averaging for a sensor for a detailed embodiment of the apparatus, system and method. This relates to mean calculation and moving average calculation. This two-step process. Step 1 provides for the calculation of vertical (column wise) and horizontal (row wise) means of current filtered data. In step 2 the current and previous mean data is 'window averaged'. The number of windows (based on order) can vary and can have weights (weighted average) for optimum performance. An averaged horizontal data is used for direction finding and used for subsequent steps. An averaged vertical data is used to determine safest height of the medical device 50 (X-ray tube head/X-ray detector) and is used directly to set the X-ray tube head height/X-ray detector height.

The geometric means of all the individual sensors are joined or "stitched" together as a matrix to create a single matrix for the whole 90 degree vision of the tube head's direction of motion (for example stitching of sensor group 1, 2 and 3 as seen in Fig. 14).

The matrix for the geometric Mean (rows data) for each sensor has 8 indices, stitching essentially means combing these matrices of 3 sensors in a single matrix of 24x8 length (24 horizontally - used for variable threshold distancing, and 8 vertically - used for positioning height to highest value).

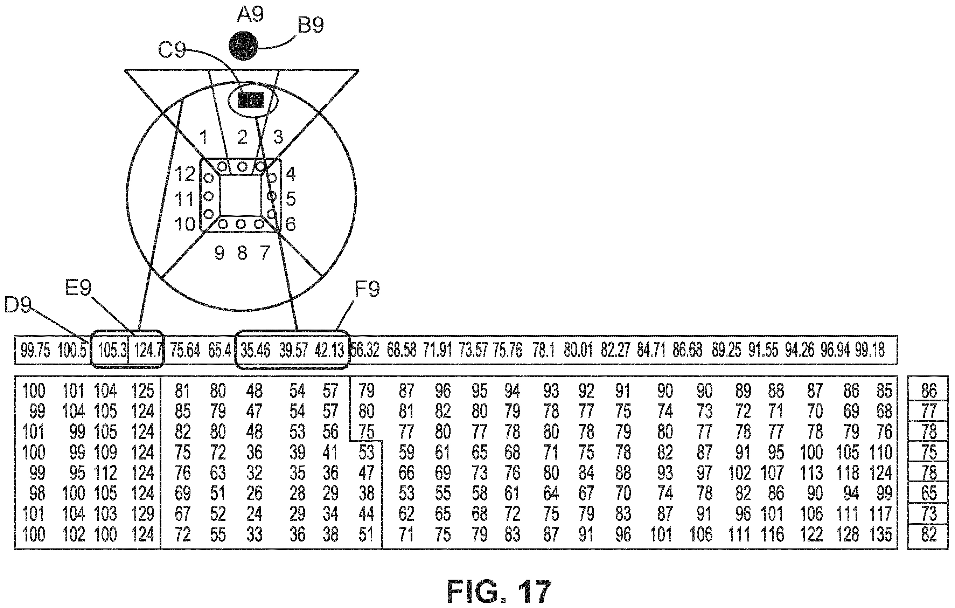

Figs. 17-19 then provide further details on the autonomous movement provided.

In Fig. 17 field of view is indicated by "A9", destination or required location is indicated by "B9", object in path is indicated by "C9", free space is indicated by "D9", threshold distance is indicated by "E9", object sensed in stitched matrix is indicated by "F9".

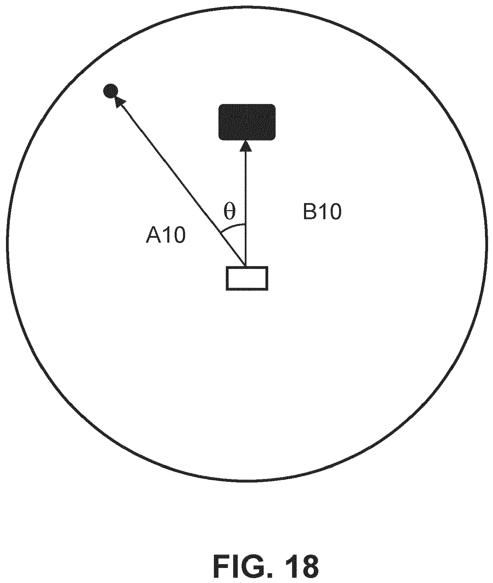

In Fig. 18 threshold distance is indicated by "A10", distance to obstacle is indicated by "B10".

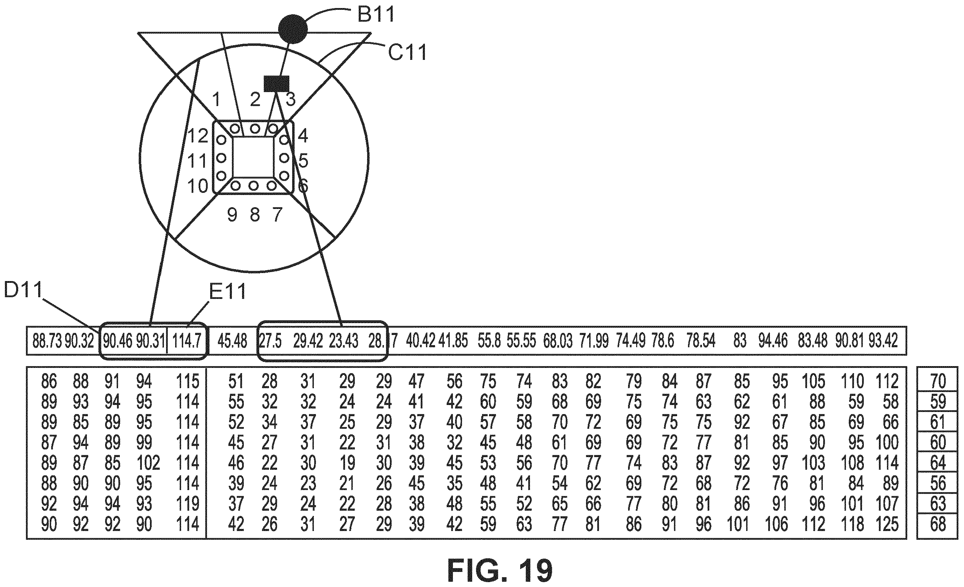

In Fig. 19 destination is indicated by "B11", object in motion path is indicated by "C11", free space is indicated by "D11", and threshold distance is indicated by "E11".

The geometric mean data provides a means of determining possible obstacles to the medical device 50 (e.g. X-ray tube head) as well means of finding maximum free space or collision free distance from the X-ray tube head. After obtaining the geometric means, the algorithm then looks at finding 'maximum free space' which essentially is the direction of the X-ray tube head/X-ray detector on the least collision risk trajectory. The system then computes a direction towards the maximum geometric mean point (124.7 as seen in Fig. 17) and sets a course towards the angle.

The angle of new trajectory is computed using the delta of indices as discussed below.

The 90 degrees field of view of the 3 sensor elements as shown in Fig. 17 is divided in 24 indices. This means that every pixel "sees" and angle = 90/24 Degree = 3.75 Degrees. The centre of the field of view is directed towards the destination (required location).

A course correction angle calculation is calculated. As shown in Fig. 17 the least distance 35.46 is at index number 7, and the threshold distance 124.7 is at index number 4. The difference = 4-7 = (-) 3. Therefore, the course correction angle = (-) (3.75^{∗}3) = (-) 11.25 Degrees. This is shown in Fig. 18.

Next, the medical device 50 (X-ray tube head/X-ray detector) moves to the threshold distance with the correction angle as shown in Fig. 19. At this new position, the obstacle is closer to the X-ray tube head/X-ray detector, but X-ray tube head/X-ray detector is on track to move away and out of line of sight of the obstacle. The new threshold distance is 114.7, and the new course correction angle is calculated. The least distance 23.43 is at index number 9, and the threshold distance 95.31 is at index number 5. The difference = 5-9 = (-) 4, providing a course correction angle = (-) (3.75^{∗}4) = (-) 15 Degrees. Likewise, new threshold distance are set, and course correction keeps on happening until obstacles are overcome and the end destination is reached.

It is to be noted that as the medical device 50 (X-ray tube head/X-ray detector) moves towards the end destination (required location), the line of sight towards the end destination changes, hence the sensing front end shall accept data from sensors neighboring the line of sight. For example, from the examples above, the next sensor group may be sensors 2,3,4, but by the time the X-ray tube head/x-ray detector reaches the end destination the sensor group may have sensors 4,5,6. Essentially, the number of sensors used for computation always remain 3. But which group is selected by algorithm depends on the line of sight towards end destination. =

In another exemplary embodiment, a computer program or computer program element is provided that is characterized by being configured to execute the method steps of any of the methods according to one of the preceding embodiments, on an appropriate apparatus or system.

The computer program element might therefore be stored on a computer unit, which might also be part of an embodiment. This computing unit may be configured to perform or induce performing of the steps of the method described above. Moreover, it may be configured to operate the components of the above described system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A medical device movement control apparatus (10), comprising:
- an input unit (20);
- at least one distance sensor (30); and
- a controller (40);
wherein the input unit is configured to receive a required location of the medical device;
wherein the input unit is configured to provide the required location of the medical device to the controller;
wherein the at least one distance sensor is configured to be mounted to a medical device (50) or is configured to be integrated with the medical device;
wherein the at least one distance sensor is configured to acquire distance data from the medical device for a plurality of angular directions about the medical device;
wherein the at least one distance sensor is configured to provide the distance data for the plurality of angular directions about the medical device to the controller; and
wherein the controller is configured to control a movement system (60) of the medical device to move the medical device from an initial location of the medical device to the required location along a route between the initial location and the required location that maintains a distance of at least a guard range distance between the medical device and one or more of objects comprising utilization of distance data for a subset of the plurality of angular directions acquired at a plurality of locations along the route.

2. The apparatus according to claim 1, wherein the controller is configured to control the movement system of the medical device to bring the medical device to a halt if distance data for an object indicates that it is closer to the medical device than the guard range distance comprising utilization of distance data for the plurality of angular directions about the medical device at one or more of the plurality of locations along the route.

3. The apparatus according to any of claims 1-2, wherein the distance data from the medical device acquired by the at least one sensor for the plurality of angular directions comprises distance data up to a threshold distance range that is greater than the guard range distance and less than a maximum detection range of the at least one distance sensor.

4. The apparatus according to claim 3, wherein the threshold is 0.5m, 1m, 1.5m, 2m, 2.5m, 3m, 3.5m.

5. The apparatus according to any of claims 1-4, wherein the plurality of angular directions comprises a plurality of angular directions in a horizontal plane.

6. The apparatus according to any of claims 1-5, wherein the subset of the plurality of angular directions at each of the plurality of locations along the route comprises angular directions over an angular range either side of a direct line between the medical device and the required location at each of the plurality of locations.

7. The apparatus according to claim 6, wherein the angular range either side of the direct line between the medical device and the required location at each of the plurality of locations comprises an angle in the horizontal plane of 10 degrees, 15 degrees, 20 degrees, 25 degrees, 30 degrees, 35 degrees, 40 degrees, 45 degrees, 50 degrees, 55 degrees, 60 degrees.

8. The apparatus according to any one of claims 6-7, wherein the controller is configured to utilize the distance data for the angular directions over the angular range either side of the direct line between the medical device and the required location at each of the plurality of locations along the route to determine a next part of the route between the initial location and the required location, and wherein the next part of the route at a location of the plurality of locations is in a direction other than the direction of the direct line between the medical device and the required location at that location if the distance data indicates that there is an object in the direction of the direct line between the medical device and the required location.

9. The apparatus according to claim 8, wherein the direction of the next part of the route at the location of the plurality of locations is determined as an angular correction to the direction of the direct line between the medical device and the required location at that location calculated as an angle between a direction towards the object and a direction towards a distance mean point.

10. The apparatus according to claim 9, wherein the distance mean point is determined from the distance data of the subset of the plurality of angular directions over the angular range either side of the direct line between the medical device and the required location at that location that results in a maximum distance value from the distance data for the subset of the plurality of angular directions over the angular range either side of the direct line between the medical device and the required location.

11. The apparatus according to claim 10, wherein the maximum distance value is determined from a summation of distance data from at least two adjacent angular directions.

12. The apparatus according to any of claims 10-11, wherein the maximum distance value is determined from the distance data of the subset of the plurality of angular directions over the angular range either side of the direct line between the medical device and the required location at that location that have been weighted, and wherein a magnitude of a weighting value decreases as the angular direction increases away from the direct line between the medical device and the required location at that location.

13. A medical device movement control system (100), comprising:
- an input unit (20);
- a medical device (50);
- movement system (60) of the medical device;
- at least one distance sensor (30); and
- a controller (40);
wherein the input unit is configured to receive a required location of the medical device;
wherein the input unit is configured to provide the required location of the medical device to the controller;
wherein the at least one distance sensor is mounted to or integrated with the medical device;
wherein the at least one distance sensor is configured to acquire distance data from the medical device for a plurality of angular directions about the medical device;
wherein the at least one distance sensor is configured to provide the distance data for the plurality of angular directions about the medical device to the controller; and
wherein the controller is configured to control the movement system of the medical device to move the medical device from an initial location of the medical device to the required location along a route between the initial location and the required location that maintains a distance of at least a guard range distance between the medical device and one or more of objects comprising utilization of distance data for a subset of the plurality of angular directions acquired at a plurality of locations along the route.

14. A medical device movement control method (200), comprising:
receiving (210) by an input unit a required location of the medical device, and wherein at least one distance sensor is mounted to or integrated with the medical device;
providing (220) by the input unit the required location of the medical device to a controller;
acquiring (230) by the at least one distance sensor distance data from the medical device for a plurality of angular directions about the medical device;
providing (240) by the at least one distance sensor the distance data for the plurality of angular directions about the medical device to the controller; and
controlling (250) by the controller a movement system of the medical device to move the medical device from an initial location of the medical device to the required location along a route between the initial location and the required location that maintains a distance of at least a guard range distance between the medical device and one or more of objects comprising utilizing distance data for a subset of the plurality of angular directions acquired at a plurality of locations along the route.

15. A computer program element for controlling an apparatus according to any of claims 1-12 or a system according to claim 13 which when executed by a processor is configured to carry out the method of claim 14.
